# EUROPEAN PATENT SPECIFICATION

(11) **EP 4 034 062 B1**
(45) Date of publication and mention of the grant of the patent: **28.08.2024**
(21) Application number: 20785460.5
(22) Date of filing: 25.09.2020
(51) Int. Cl.: A61F 13/20

(54) **TAMPON PRESS AND SHAPED TAMPON**
TAMPONPRESSE UND GEFORMTER TAMPON
PRESSE À TAMPON ET TAMPON FAÇONNÉ

(30) Priority: 27.09.2019 US 201962907215 P; 27.09.2019 US 201962907153 P
(43) Date of publication of application: 03.08.2022
(73) Proprietor: Johnson and Johnson GmbH, 41470 Neuss (DE)
(72) Inventor: BUSCHHAUS, Mirko, 42289 Wuppertal (DE); KIMBALL, David L., Flemington, New Jersey 08822 (US); ROBBE, Lionel, 42289 Wuppertal (DE); BROOKS, Nick, Warwick Warwickshire CV34 6UQ (GB); MORGAN, Peter, Leamington Spa Warwickshire CV32 6NQ (GB); STEMBRIDGE, James, Coventry Warwickshire CV5 6GW (GB)
(74) Representative: Carpmaels & Ransford LLP
(86) International application number: PCT/EP2020/076935
(87) International publication number: WO 2021/058755

(56) References cited:
- EP-A1- 1 485 053
- WO-A1-2018/220587
- US-A1- 2005 113 787

## Description

### Field of the invention

The present invention relates to an intravaginal tampon, such as an absorbent of catamenial tampon, for feminine hygiene. In particular, it relates to a press for producing such tampon having penetrating dies with shaped pressing faces; the tampons made therewith; and a digital tampon having a shaped profile providing a variable cross section.

### Background of the invention

Intravaginal tampons have been used for decades. Two types of tampons are present on the market, digital tampons and tampons requiring an applicator. Typically, digital tampons are formed by radial compression of a blank with a set of jaws while applicator tampons are formed by molding a blank. Both types of tampons have a generally cylindrical shape and both have advantages and disadvantages.

Digital tampon can be inserted manually without an applicator. They have a neat smooth surface; however, the compression column created by the press jaw within the tampon pledget to generate the rigidity required for digital insertion may be uncomfortable for some consumers. Applicator tampons may not have this rigidity issue; however, they require the use of an applicator and generate additional waste.

It is also important to consider that a tampon may change in position while in place depending on the activity of the wearer, e.g. moving, doing sports or even coughing. This can create discomfort and even leakage if the new position of the tampon is not adapted.

Leakage avoidance and wearing comfort are two important benefits for the consumer.

Shaped tampons have been proposed to fulfill these requirements. These existing shaped products are claimed to match more precisely the wearer's anatomy and thus provide better protection against bypass leakage, reduce discomfort and generally stay in place more efficiently within the vagina.

EP1267782B1 by the Procter & Gamble Company discloses a cylindrical tampon that radially expands into an oval cross section shape when exposed to a wet environment. The tampon is rolled into an oval cross section blank which is compressed by a press jaw into a cylindrical tampon.

US20140265026A1 by Eveready Battery Company discloses a tampon with spirally shaped longitudinal grooves and the press jaw for shaping said tampon.

US8574210B2 by Ontex Hygieneartikel Deutschland discloses a tampon with longitudinal groves and a press jaw for shaping it.

EP1759678A1 by Ontex Hygieneartikel Deutschland discloses a tampon with longitudinal groves and a press jaw for shaping a mushroom shaped tampon.

EP2900467A1 by Kimberly-Clark Worldwide discloses a press jaw for tampon forming. The press jaw is designed to decrease the area of the compression surface in contact with the tampon as the press moves inward.

US9622919B2 by Johnson & Johnson GmbH discloses a process for forming a shaped tampon with a radial forming process starting from an intermediate blank. The semi-finished tampon pledget is pushed into a mold to obtain its final shape through expansion. Several alternative shapes are disclosed. The compressed pledget may contain grooves but US9622919B2 is silent about the effect of controlled expansion on grooves.

EP2712594B1EP, EP2712595B1 & EP2712596B1 by Johnson & Johnson GmbH disclose tampons with deep intersecting grooves; said grooves are continuous in EP2712595B1, detached in EP2712596B1 or could represent 150% of the pledget length in EP2712594B1. EP2712594B1 further discloses an embodiment with post-processing steps in which grooves are formed with a finishing mold or calendering rollers. The disclosed tampons are cylindrical and have a circular cross section. Also disclosed is a tampon press with sets of adjacent jaws passing through the same press space during manufacture. The press jaws comprise two sets of penetrating dies, each set having a penetrating face corresponding to a groove segment shape.

EP1680062A1, EP1686941A1, EP1485053B1, EP1485054B1 and EP1485055B1 by Procter & Gamble disclose tampons with a serpentine-shaped outer surface. The tampon has an insertion end region; a withdrawal end region; and a center region. The insertion end region has an insertion end fiber density. The withdrawal end region has a withdrawal end region fiber density. The center region has a center region fiber density. The insertion end region fiber density is greater than the center region fiber density. The shaped tampons disclosed have varying average fiber density regions. Groove patterns are not discussed and tampons with a non-circular cross section are not disclosed.

US8684987B2 by Procter & Gamble discloses a self-orienting tampon having a non-circular cross-section. The tampon includes a self-sustaining, fluid-expanding, compressed absorbent pledget having one or more absorbent materials. The tampon has a width, a thickness and a length. The width can be greater than the thickness and an aspect ratio of the width to the thickness can be from greater than about 1.4:1 to less than about 2.0:1. Neither a groove pattern nor a curved profile of the outer surface along the longitudinal length is disclosed.

US20040199137A1 by Peter J. B. Lamb discloses a tampon which includes an elongate absorbent body which is non-circular in outline in end view. US20040199137A1 further discloses a tampon which has an angular orientation relative to its longitudinal axis. Neither a groove pattern nor a curved profile of the outer surface along the longitudinal length is disclosed.

US8474114B2 by Ruggli Projects discloses a method for a shaping step completing a process for producing a tampon which is produced of an absorbent material. At least one groove is embossed onto the peripheral surface by radially compressing at least one region of the tampon extending along a peripheral surface of the tampon, the groove plane substantially extending normal to the longitudinal extension of the tampon. Cross section perimeter and outer surface profile are not discussed. Longitudinal groove patterns are not disclosed.

US5370633A by Josue J. Villalta discloses an anatomically shaped tampon. The main body has a pair of side ridges connected to an intermediate wall forming an external configuration resembling a capital H. One end of the tampon includes an outwardly opening cavity to fittingly receive the uterine cervix, limiting movement of the tampon. Its cross-section remains the same along the tampon pledget length. Particular cross-sections and outer surface profiles are disclosed. Groove patterns are not mentioned.

WO 2018/220587 A1 discloses an apparatus for manufacturing an intravaginal tampon for feminine hygiene including a tampon press having a plurality of elongate press dies disposed about a central press axis to form a press cavity and a cylindrical carrier having a diameter less than that of the predetermined finished diameter.

While some of the above examples describe presses to produce tampons with different profiles or groove configurations, these tampons have traditional cylindrical shapes.

Further, the above examples fail to provide indications about the press configuration required to achieve a tampon with a complex longitudinal or radial shape.

Despite all the shaped tampons proposed in the prior art, there is still a need to address the major concerns of the consumers: wearing comfort and leakage avoidance.

### Summary of the invention

The present invention is proposed to solve these problems by providing shaped tampons that are adapted to the feminine anatomy and shows good fluid absorption properties.

It has been discovered that a shaped tampon with a shaped longitudinal profile and an elliptical cross section on at least a part of its length can provide a better fit to the wearer's anatomy and thus increases the wearer's comfort without compromising the fluid absorption properties.

The present invention is defined in the appended claims.

### Brief description of the drawing

Fig.1 is a side view of three examples of penetrating dies. For each example, a pair of dies is shown. Each example has a different shaped profile.
Fig. 2 is a radial view of a press comprising eight penetrating dies. By radial view it is meant according to a plane perpendicular to the main longitudinal axis.
Fig. 3 is a perspective view of a press comprising eight penetrating dies and a compressed tampon. The penetrating dies are in an open configuration. For clarity, the grooves have been omitted from the tampon.
Fig. 4 is a perspective view corresponding to Figure 3, where the penetrating dies are in a closed configuration. For clarity, two penetrating dies have been omitted so that the tampon is visible.
Fig. 5 is a perspective view of a split composite penetrating die. The front part is on the right side of the view and the back part is on the left side of the view. The pressing faces are pointing upward.
Fig. 6A is a perspective view of eight composite penetrating dies, arranged radially, in an open configuration.
Fig. 6B is a perspective view corresponding to Figure 6A, showing only the front parts of the composites penetrating dies.
Fig. 6C is a perspective view corresponding to Figure 6A, showing only the back parts of the composites penetrating dies.

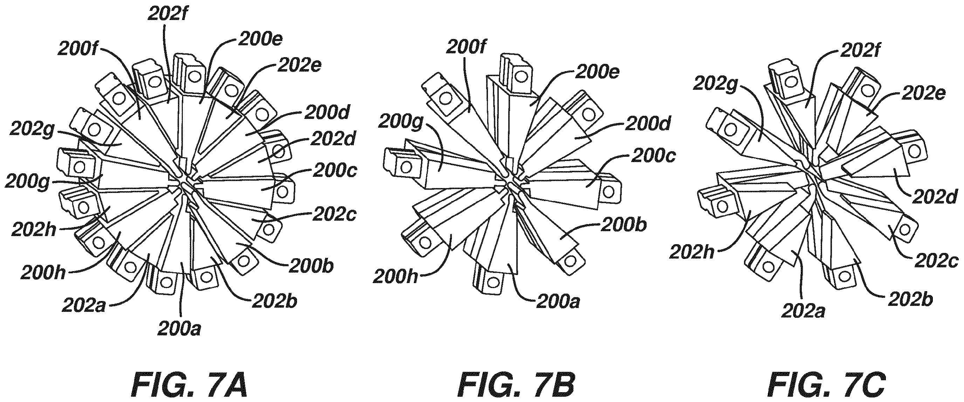

Fig. 7A is a perspective view of eight composite penetrating dies, arranged radially, in a closed configuration.
Fig. 7B is a perspective view corresponding to Figure 7A, showing only the front parts of the composites penetrating dies.
Fig. 7C is a perspective view corresponding to Figure &A, showing only the back parts of the composites penetrating dies.
Fig. 8 is a perspective view of a composite penetrating die engaging with a tampon. Both front part and back part of the composite penetrating die are in a closed configuration, creating a substantially continuous groove made of two segments.
Fig. 9 is a perspective view of two back parts of a composite penetrating dies engaging with a tampon.
Fig. 10 is a perspective view of the front parts of two adjacent composite penetrating dies and the back part of one composite penetrating die positioned to produce a bifurcated groove on the outer surface of an absorbent tampon pledget. The composite penetrating die back part is represented at the forefront of the drawing and colored in grey.
Figure 11 is an illustration of a series of 6 tampons according to one embodiment the present invention. Tampons are identified by a letter, from A to G, from the left to the right side of the picture.
Figure 12 is side view of a wrapped tampon.
Figure 13 is front plan view of the tampon in an unwrapped configuration with a withdrawal string extending therefrom.
Figure 14 is a drawing representing an axial cross-section view of the insertion portion of a tampon according to the present invention.
Figure 15 is a drawing representing an axial cross-section view of the withdrawal portion of a tampon according to the present invention.
Figure 16 is a drawing representing a superposition of the axial cross section views of the insertion portion and the withdrawal portion of a tampon according to the present invention.

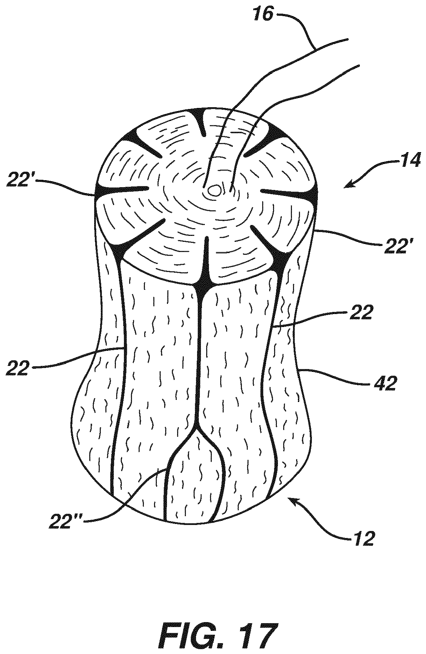

Figure 17 is a drawing representing a perspective view of a tampon according to the present invention. Its insertion tip is pointing backward and down; and its withdrawal end is in the foreground. Its withdrawal string is also represented, protruding from the withdrawal end.

### Detailed description of the preferred embodiments

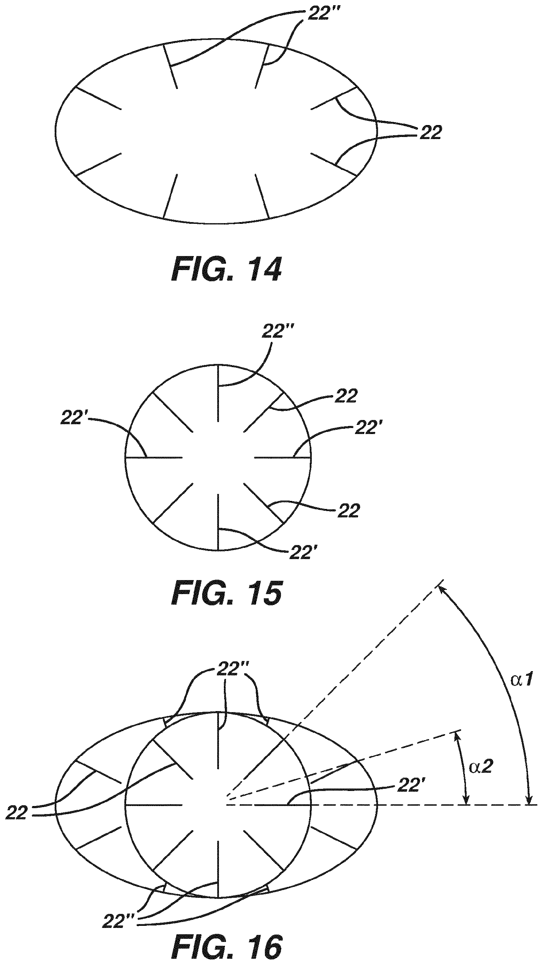

As used in the specification and the claims:
the term "shaped longitudinal profile" and variants thereof relate to the configuration of the edge of an object, considered along the longitudinal axis of the press;
by "shaped" it is meant that the edge of the object is not straight on its full length; it may be curved, possibly several times. In other words, the shaped profile may comprise concave or convex curves, alone or in a succession with one or more curves. It may also comprise one or more straight portions in association with one or more curved portions;
the term "tampon blank" and variants thereof relate to an uncompressed tampon material;
the term "pledget" and variants thereof relate to a pad or a compress of absorbent material such as fibers designed to absorb bodily fluids. A "tampon pledget" or "absorbent tampon pledget" relates to the compressed absorbent material after compression of a tampon blank in a press;
the term "groove" and variants thereof relate to an indentation in the surface of the tampon pledget. For clarification, grooves may be "penetrating grooves", extending at least 0.7mm (or 10% of the radius, whichever is greater) into the tampon pledget or they may be "shallow grooves", primarily surface indentations without significant penetration (of no more than 0.7mm, not more than 10% of the radius) into the tampon pledget body. Regions between grooves may take the form of ribs;
the term "adjacent" and variants thereof relate to elements that are positioned side by side but not touching each other. It is preferable to have the extremities not touching each other as it would create friction and heat and it might be detrimental for the proper operating of the press at high speed;
the term "extremities" and variants thereof relate to the end of the pressing faces according to their narrow longitudinal dimension;
the term "bifurcated", and variants thereof relate to a line that divides into two parts or branches such as in a "Y" shape;
the term "radial plane" and variants thereof relate to a plane perpendicular to the main longitudinal axis of the press;
the terms "partially disengaging" a group of penetrating dies mean that the said dies remain engaged with the front portion of the compressed tampon pledget but may retract in a way sufficient to allow the ejecting device, for example an ejection rod, to push the compressed tampon pledget outside the press without having said ejection device touching the penetrating dies;
the longitudinal axis X-X of a shaped tampon is defined as the axis passing by the insertion tip and the center of the withdrawal end;
an axial cross-section is defined as a plane perpendicular to the said longitudinal axis X-X, shown in Figures 14-16;
a longitudinal cross section is defined as a plane including the longitudinal axis;
an ellipse can be defined mathematically as a closed conic section shaped like a flattened circle and formed by an inclined plane that does not cut the base of the cone. An elliptical contour may be as shown on Figure 14;
the term "maximal axial cross section" of the insertion portion is defined as the axial cross section for which the perimeter of the insertion portion reaches its maximal value;
the term "minimal diameter" is the distance between the two points corresponding to the intersections of the ellipse minor axis with the ellipse's contour;
the term "maximal diameter" is the distance between the two points corresponding to the intersections of the ellipse major axis with the ellipse's contour;and
by "large side" it is meant the side parallel to the major axis of the elliptical cross section of the insertion portion as defined above. A press is disclosed for information for shaping an absorbent tampon blank into a shaped tampon pledget has a central cavity, the main longitudinal axis of the press passing in the middle of the central cavity; a plurality of penetrating dies arranged radially around the said main longitudinal axis; and at least one cam to actuate the plurality of penetrating dies. The central cavity is arranged and configured to accommodate an absorbent tampon blank having a front portion and a back portion. Each penetrating die has a pressing face on at least a fragment of its side facing the central cavity. At least one penetrating die has a pressing face with a first shaped longitudinal profile and at least one penetrating die has a pressing face with a second shaped longitudinal profile. The front portion corresponds to the insertion tip of the tampon pledget and the back portion corresponds to the withdrawal end of the tampon pledget. The first shaped longitudinal profile and the second shaped longitudinal profile may be different.

Having shaped longitudinal profiles on the penetrating die pressing faces enables the production of a tampon pledget with a shaped profile. When several different longitudinal profiles are used, it becomes possible to create tampon pledgets with complex longitudinal profiles and potentially non-circular cross-section on at least a portion of the tampon pledget. These shaped tampons are believed to provide enhanced comfort to the user by having a shape more adapted to the vaginal configuration, compared to straight cylindrical tampons.

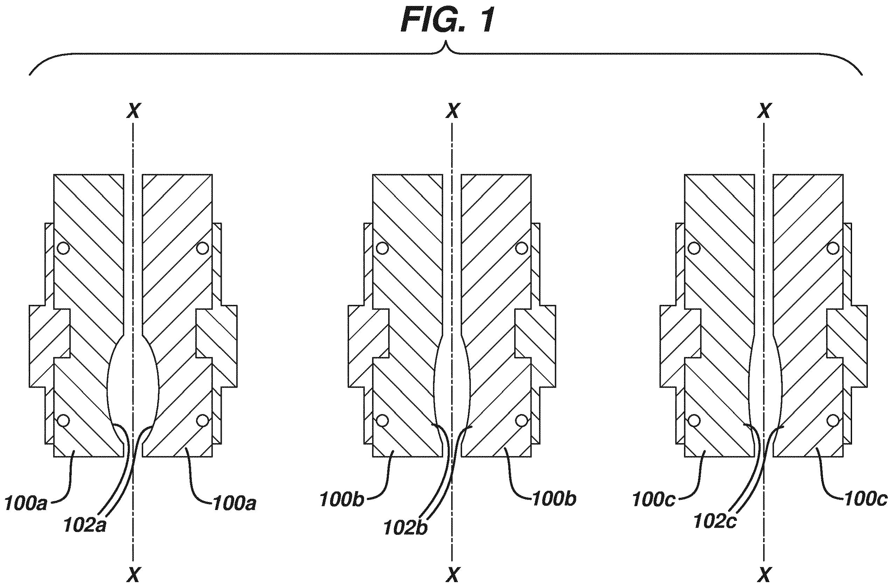

Figure 1 illustrates three pairs of penetrating dies 100a, 100b, 100c with their pressing faces 102a, 102b, 102c having three different shaped profiles of said pressing face pairs.

The plurality of penetrating dies may be arranged about the main longitudinal axis X-X in opposing sets of penetrating dies. In that configuration, at least one opposing set of penetrating dies may be arranged to have the opposing pressing faces disposed at 180° according to the main longitudinal axis.

At least one opposing sets of penetrating dies may be arranged to have two pressing faces opposed to one pressing face; or as an alternative, to have one pressing face moving toward a gap between two other pressing faces and opposing sets of penetrating dies may comprise an even or odd number of penetrating dies.

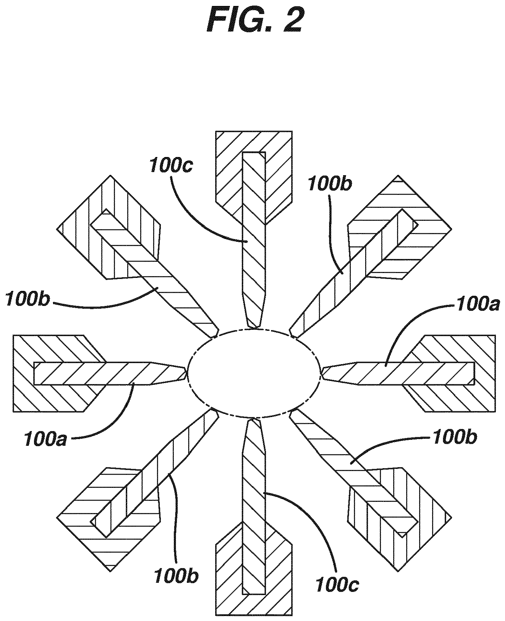

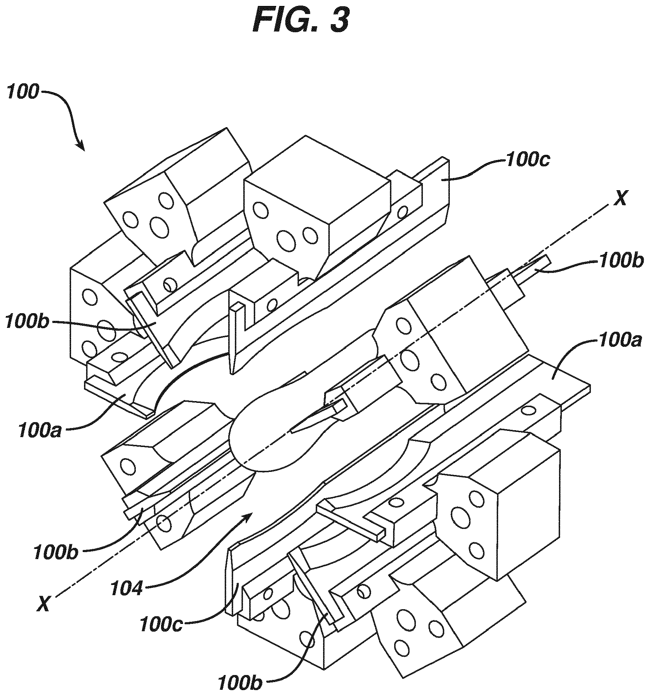

Referring to Figures 2 and 3, three opposing sets of penetrating dies are represented radially arranged around the central cavity 104 of the press 100. A first opposing set of two penetrating dies 100a has a large maximum gap between opposed pressing faces 102a, a second opposing set of four penetrating dies 100b has an intermediate maximum gap between opposing pressing faces 102b and a third opposing set of two penetrating dies 100c has the smallest maximum gap between opposing press faces 102c (as shown in Figure 1).

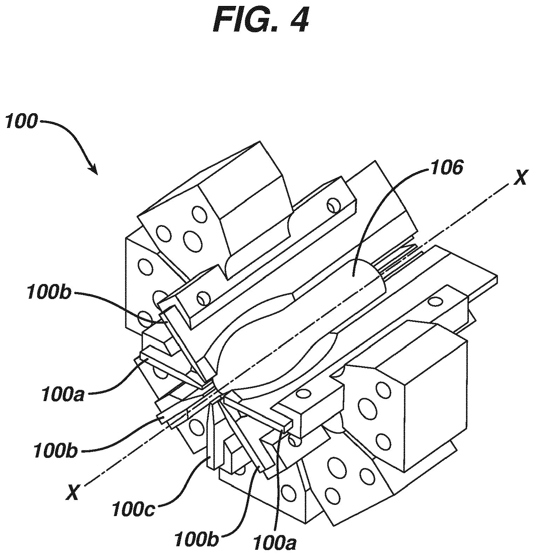

Figure 4 corresponds to Figure 3, with the penetrating dies engaged in the tampon pledget 106 along the longitudinal axis X-X. For clarity, two penetrating dies have been omitted from Figure 4.

Preferably, at least one opposing set of penetrating dies may have an even number of penetrating dies. In a further preferred configuration, at least one opposing set of penetrating dies may have at least four penetrating dies disposed around the main longitudinal axis.

The press may have an even number of opposing sets of penetrating dies. As illustrated in Figures 1-4, the press may have at least 3 different opposing sets of penetrating dies.

In a preferred configuration, within an opposed set of penetrating dies, the groups of opposed pressing faces may be identical. In a further preferred configuration, such as illustrated in Figures 1-4, the pressing faces of an opposed set of penetrating dies may have axial symmetry. In other words, within a preferred configuration, the pressing faces of said opposed set of penetrating dies may have at least one plane of symmetry, preferably two planes of symmetry.

In an embodiment of the invention, a press for shaping absorbent tampon blank incorporates penetrating dies that are divided longitudinally, creating a group of composite penetrating dies. The composite penetrating dies have two parts: a front part and a back part. The front parts of the composite penetrating dies are grouped together and referred to as the front group. The back parts of the composite penetrating dies are grouped together and referred to as the back group.

The front group is arranged and configured to shape the insertion end of the tampon.

The back group is arranged and configured to shape the withdrawal end of the tampon.

The use of composite penetrating dies enables the creation of complex shaped tampons as the penetrating dies shaping the front portion and the back portion of the tampon blank may be different and can be operated in an non-simultaneous way. This may be relevant as the central cavity has a limited space and would only provide the possibility to operate a limited number of pressing faces at the same time.

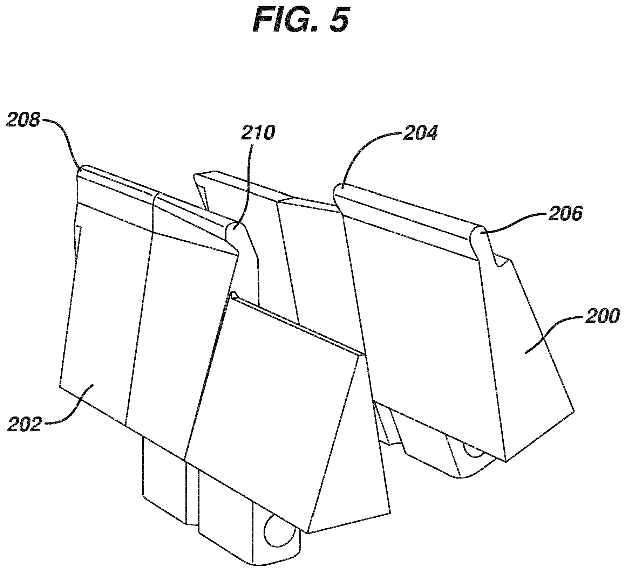

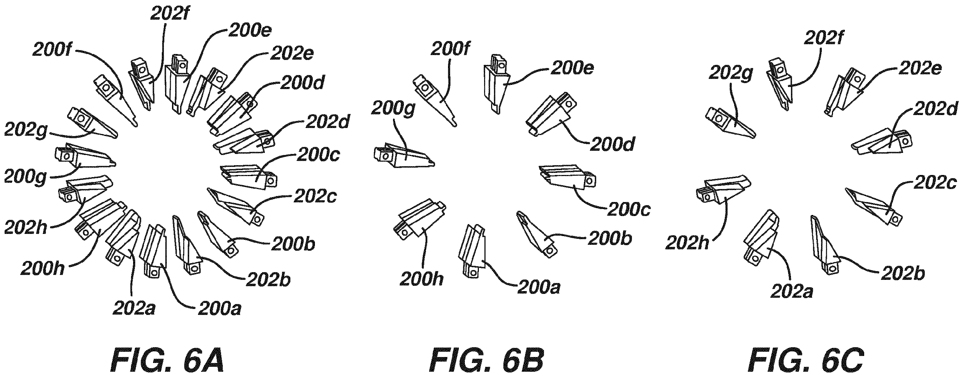

Referring to Figure 5, an embodiment of a composite penetrating die comprising a front part 200 and a back part 202 is shown. As shown in Figures 6A, 6B and 6C, all the composite penetrating dies front parts 200a-200h will constitute the front group (or composite penetrating dies front group) such as illustrated in Figure 6B. Accordingly, all the composite penetrating die back parts 202a-202h will constitute the back group (or composite penetrating dies back group) such as illustrated in Figure 6C. Figure 6A discloses how the front group and back group may be arranged along the longitudinal axis.

The above-described configuration of penetrating dies allows the front group and the back group to be actuated independently. This particular configuration allows more freedom in the operation of the press according to the invention and might be achieved, for example, by a first cam operating the front group and a second cam operating the back group.

In an embodiment, all penetrating dies may be composite penetrating dies.

In an embodiment, at least two composite penetrating die front parts are identical, e.g. 200c and 200g. Again, the composite penetrating die front parts may be arranged by opposing sets.

In an embodiment, a press according the present invention may also comprise at least two composite penetrating die back parts that are identical, e.g. 202a and 202e. Again, the composite penetrating die back parts may be arranged by opposing sets.

In an embodiment, the press has at least two of the composite penetrating dies that have a back part and front part pressing faces that are positioned to produce, on the outer surface of an absorbent tampon pledget, a substantially continuous groove made of two segments; the extremities of the two pressing faces being adjacent at a connection point.

Referring to Figure 5, in this embodiment, the two extremities 204, 206 of a composite penetrating die front part 200 and the two extremities 208, 210 of a composite penetrating die back part 202 are indicated. Each pressing face would have two types of extremities: exterior extremities 206, 208 of the front part and back part, respectively, and interior extremities 204, 210. The extremities considered in the present embodiment to form a substantially continuous groove are the two interior extremities 204, 210 as these act on the tampon blank and define a connection point of the ensuing groove.

The connection point may be located approximately in the central portion of the tampon blank. By central portion it is meant 25% to 75% of the length of the tampon pledget, preferably 40% to 60% of the length of the tampon pledget; wherein the length is considered according to the longitudinal axis.

In a particular embodiment, the press has a plurality of composite penetrating die back parts that have pressing faces that are not parallel to the main longitudinal axis and preferably the pressing faces are helically arranged. This configuration increases the length of the grooves, thereby increasing the path of any fluid along the tampon and thus decreasing the risk of leakage.

In an alternative embodiment, all composite penetrating die front parts may have pressing faces that are parallel to the main longitudinal axis. This configuration may reduce the ejection forces necessary to remove the pledget from the press.

In another embodiment, a press according to the present invention may have at least two adjacent composite penetrating dies having front part and back part pressing faces that have no connection point. Thus, at least two composite penetrating dies may have front part and back part pressing faces that would not produce a substantially continuous groove on the outer surface of the tampon pledget.

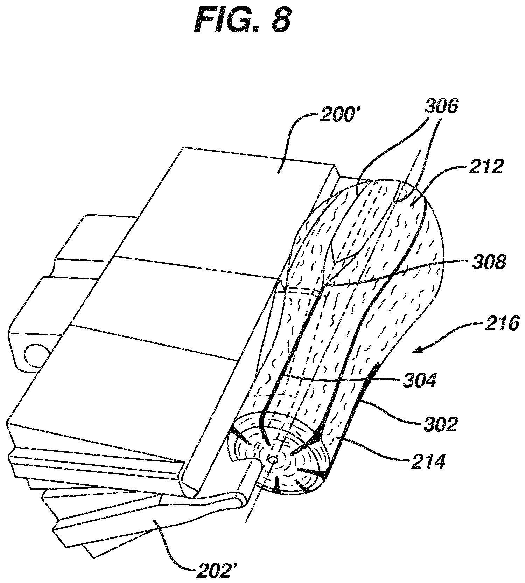

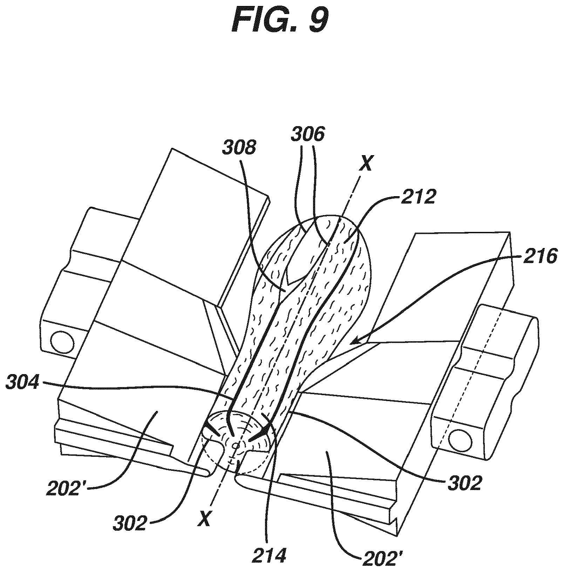

Referring to Figures 8 and 9, two composite penetrating die back parts 202', opposed to each other across the longitudinal axis X-X, are engaged in a tampon pledget 206. The groove 302 created by the two composite penetrating die back parts 202' are located on the tampon 212 back portion 214a and have no continuity with any grooves located on the tampon front portion. They simply terminate in the central portion 216 of the tampon 212.

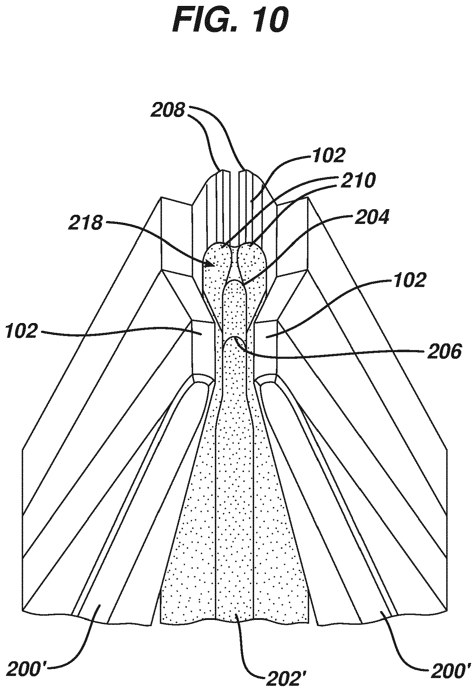

Referring to Figure 10, the pressing faces 102 of at least two adjacent composite penetrating die front parts 200' (also shown in Figures 8 and 9) and the pressing face of one composite penetrating die back part 202' (also shown in Figures 8 and 9) may be positioned to produce, on the outer surface of an absorbent tampon pledget, a bifurcated groove (shown in Figures 8 and 9) made of three segments: a back part groove segment 304 and two front part groove segments 306; the interior extremities of the three pressing faces are adjacent at a connection point 308 of the groove and in a location identified at cross-over point 218. In more detail, three penetrating dies: one penetrating die back part 202' and two penetrating die front parts 200' are shown in a closed position. Pressing face 102, extending between extremities 204, 206 of the composite penetrating die back part 202', is arranged and configured to form the withdrawal end portion 304 of the bifurcated groove (see Figure 8) and the pressing faces 102 extending between the two extremities 208, 210 of two associated composite penetrating die front parts 200' are arranged and configured to form the insertion end portion 306 (see Figure 8) of the bifurcated groove.

A press according the present invention may comprise at least 2 sets of composite penetrating dies positioned to have their pressing faces producing at least two of said bifurcated grooves on the outer surface of an absorbent tampon pledget.

In yet another embodiment of the present invention, the press may comprise at least two, preferably four, preferably six and most preferably eight, of the composite penetrating die front parts having pressing faces with a shaped longitudinal profile.

Furthermore, a press according to the present invention may have composite penetrating die front parts comprising pressing faces with at least two different shaped longitudinal profiles.

More particularly, the shaped longitudinal profile of the pressing faces may be curved, creating a concave profile on the pressing faces.

In a preferred embodiment, the present invention relates to a press, wherein the connection points of the two pressing faces of the composite penetrating dies are positioned to produce a substantially continuous groove made of two segments and the three pressing faces of the composite penetrating dies positioned to produce a bifurcated groove made of three segments are not all included in the same radial plane.

Alternatively, the present invention may relate to a press wherein the connection points of the two pressing faces of the composite penetrating dies positioned to produce a substantially continuous groove made of two segments and the three pressing faces of the composite penetrating dies positioned to produce a bifurcated groove made of three segments are all included in the same radial plane.

The position of the connection points of the different grooves that may be on the tampon pledget can have an influence on the flexibility of the tampon. In certain circumstance it might be a benefit to provide a tampon with an improved flexibility or, in other words, a tampon that could bend along its longitudinal axis. This type of tampon is believed to provide increased comfort to the wearer.

A method for producing a shaped tampon pledget according to the present invention may comprise the steps of introducing a tampon blank in a press according to the present disclosure, actuating a first group of penetrating dies to compress the tampon blank with a first set of pressing faces, actuating a second group of penetrating dies to compress the tampon blank with a second set of pressing faces, partially disengaging one of the groups of penetrating dies to move the corresponding pressing faces into a transfer position, thus allowing the front portion of a compressed tampon pledget to pass through said partially disengaged group of penetrating dies, partially disengaging the second group of penetrating dies and ejecting a resulting compressed tampon pledget from the press to a carrier device.

A preferred method for producing a shaped tampon according to the present invention may comprise the steps of introducing a tampon blank in a press according to the present disclosure, actuating the composite penetrating die front parts to compress the pledget front portion with the pressing faces of the composite penetrating dies front parts, actuating the composite penetrating die back parts to compress the pledget back portion with the pressing faces of the composite penetrating die back parts, partially disengaging the composite penetrating die back parts to release the pledget back portion from the pressing faces of the composite penetrating die back parts, partially disengaging the composite penetrating die front parts, evacuating the compressed tampon pledget from the press to a carrier device, fully disengaging the composite penetrating die front and back parts.

The composite penetrating die front parts of the present invention may be actuated in a non-simultaneous way. In other words, not all the penetrating die front parts are engaging the tampon blank at the same time. The space in the central cavity is limited and it might occur that in some configuration all the penetrating dies cannot be fully engaged with the tampon at the same time; in this case, it is required that some of penetrating dies disengage, at least partially, before other penetrating dies can compress the tampon blank. The same configuration may occur for the penetrating die back parts, namely: the composite penetrating die back parts may be actuated in a non-simultaneous way.

In yet another embodiment, the invention may refer to a shaped catamenial tampon produced with a press as described in the present disclosure.

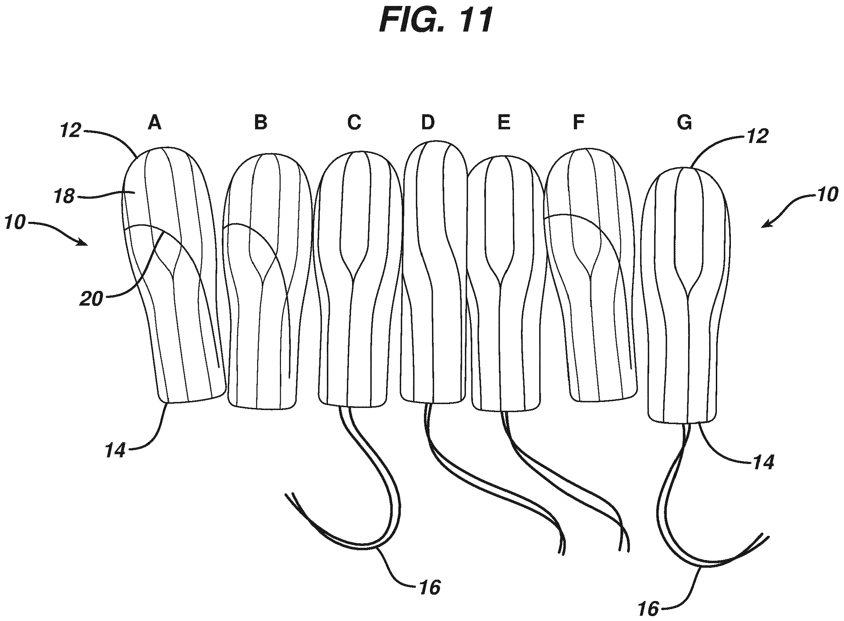

As shown in Figure 11, a shaped tampon 10 is an elongate compressed fibrous pledget extending from a rounded insertion portion 12 to a withdrawal portion 14 having a withdrawal string 16 extending therefrom. These may be enclosed in a primary packaging 18 having a line of weakness or tear line 20 to enable opening of the wrapper 20 for removal of the enclosed tampon 10. The series of tampon views illustrates the rotation of an exemplary tampon through approximately 180° and illustrates the shaping and changing cross-section dimensions around the tampon. Tampons A, B, C, E, F and G show the larger side of their insertion portion. Tampon D shows the smaller side of its insertion portion. Tampons A, B and F are wrapped in a primary packaging 18. Tear line 20 for opening the packaging are indicated by a black curved line. Tampons C, D, E and G are not wrapped. A withdrawal string 16 from each tampon is lying respectively below the withdrawal portion 14 of each unwrapped tampon 10.

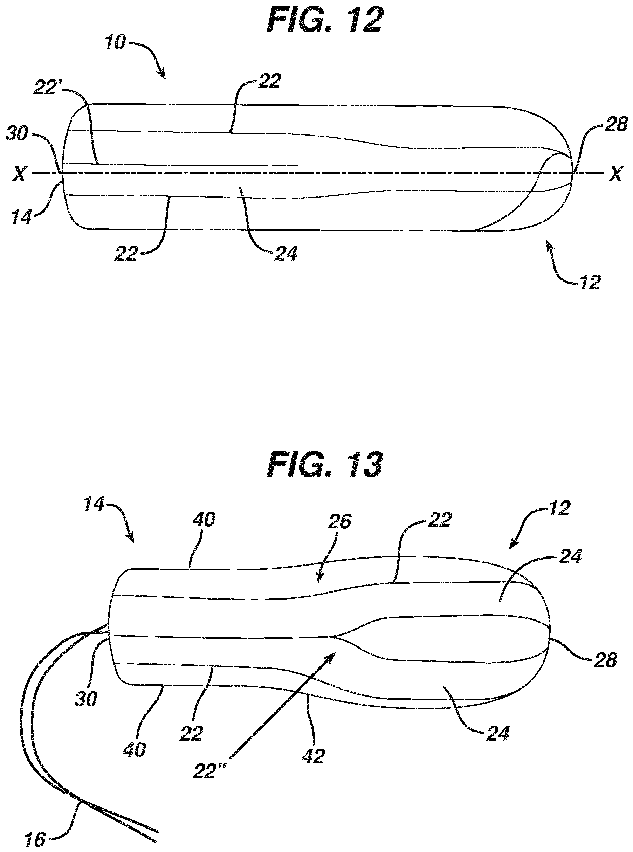

Figure 12 is side view of a wrapped tampon 10. This shows the side profile of the insertion portion 12. Figure 13 shows the front plan view of the tampon 10 showing the larger profile of the insertion portion 12. The tampon 10 of Figures 12 and 13 has a plurality of longitudinally extending grooves 22 with ribs 24 disposed therebetween. Figure 12 illustrates a truncated groove 22' and Figure 13 illustrates a bifurcated groove 22". The combination of the bifurcated groove 22" and the truncated groove 22' enables compression as described above to provide the unique elliptical cross-section of the insertion portion 12 of the present tampon 10.

The outer ellipse shape shown in Figure 14 represents the perimeter of the tampon insertion portion 12. The eight lines orthogonal to the oval perimeter represent the positions of the grooves 22 on the insertion portion 12.

The circle shown in Figure 15 represents the perimeter of the tampon withdrawal portion 14. The eight lines orthogonal to the circular perimeter represent the positions of the grooves 22 on the withdrawal portion 14.

Figure 6 is a superposition of Figures 4 and 5. Circular withdrawal portion 14 cross-section is at the center and extending outwardly on the sides is the insertion portion 12. The longitudinal axes of the two portions are registered. The positions of the grooves 22 on both insertion and withdrawal portions are represented by the lines orthogonal to either the elliptical or circular perimeters and are grouped to show the bifurcated grooves 22" and the location of the truncated groove 22'.

The present invention discloses a shaped tampon, having a longitudinal axis, an insertion portion with an insertion tip and a withdrawal portion with a withdrawal end, the insertion and withdrawal portions being connected through a central portion and each portion comprising a plurality of grooves, wherein the contour of said insertion portion 12 in an axial cross section plane is substantially elliptical with a major axis and a minor axis and the major axis is not equal to the minor axis. The insertion portion 12 has maximal axial cross-section and the contour of the insertion portion 12 is curved in at least one longitudinal cross-section plane. The grooves 22 on the insertion portion 12 extend from the central portion 26 to the insertion tip 28 and they converge toward the domed insertion tip 28.

This particular shaped profile is believed to be adapted to the vaginal anatomy and to confer a better positioning of the tampon inside the vaginal cavity as well as an easier insertion. During insertion, positioning and, if the consumer changes position, while wearing the tampon pledget, the tampon will automatically rotate along its longitudinal axis to match the vaginal wall configuration. This provides improved positioning for the user and can provide a comfort improvement over prior art tampons. The improved positioning can reduce risk of leakage, too.

Also, this particular shape of the tampon according to the present invention may create a higher expanding insertion portion on top of the tampon pledget and thus most of the absorption may occur in an area where the vagina is less sensitive, increasing the consumer wearing comfort

In another embodiment of the shaped tampon according to the present invention, the longitudinal opposite sides 40 of the contour of the withdrawal portion 14 according to a longitudinal cross-section plane are substantially parallel.

In other words, in this embodiment, the withdrawal portion of the tampon may have a contour of any shape, in an axial section plane, but said contour remains constant when the considered axial cross section plane moves along the longitudinal axis.

In another embodiment of the shaped tampon according to the present invention, the contour of the withdrawal portion according to an axial cross section plane is substantially circular.

In this embodiment, the withdrawal portion of the tampon may have a contour of circular shape, in an axial section plane, but said contour perimeter varies when the considered axial cross section plane moves along the longitudinal axis. For example, the withdrawal portion of the tampon may have an hourglass, a tapered or a hemispherical shape.

In a preferred configuration, the withdrawal portion may be substantially cylindrical.

In a preferred embodiment of the shaped tampon according to the present invention, the central portion 28 contains a shoulder 42 at which the insertion portion 12 and the withdrawal portion 14 transition.

More precisely, the shoulder may be located between 1/4 and 3/4 of the length of the longitudinal axis of the tampon.

Even more precisely, the shoulder may be located between 1/3 and 2/3 of the length of the longitudinal axis of the tampon.

The shoulder may also be located between 1/3 and 1/2 of the length of the longitudinal axis of the tampon, wherein said 1/3 designates the first third of the tampon length starting from the insertion tip 28.

In a preferred embodiment, the shoulder may be located substantially at the center of the longitudinal axis of the tampon.

It is believed that these particular proportions between the length of the insertion portion and withdrawal portion create a shaped tampon that will be particularly well adapted to the vaginal anatomy and will help prevent leakage and increase wearing comfort of the shaped tampon.

In a preferred configuration, the maximal axial cross section of the insertion portion may be located, along the longitudinal axis, between the central portion 26 and 10mm from the insertion tip 28.

Alternatively, in another preferred configuration of the present invention, the maximal axial cross section of the insertion portion 12 may be located, along the longitudinal axis, between 1 mm and 10mm from the insertion tip 28.

In a particular embodiment of the shaped tampon according to the present invention, the length of the minor axis of the maximal axial cross section of the insertion portion 12 is equal or inferior to the diameter of the withdrawal portion 14, according to an axial cross section.

It is believed that these proportions between the diameters of the insertion portion 12 and the withdrawal portion 14 may create a shaped tampon 10 that would be particularly well adapted to the vaginal anatomy and would help prevent leakage and may increase the wearing comfort of the shaped tampon 10.

In another particular embodiment of the shaped tampon according to the present invention, the minimal diameter of the maximal axial cross section of the insertion portion 12 may be at least equal to the diameter of the withdrawal portion 14, according to an axial cross section.

More particularly, the minimal diameter of the maximal axial cross section of the insertion portion 12 may be equal to the diameter of the withdrawal portion 14, according to an axial cross section.

Alternatively, the minimal diameter of the maximal axial cross section of the insertion portion 12 may be inferior to the diameter of the withdrawal portion 14, according to an axial cross section.

In another configuration of the shaped tampon 10 according to the present invention, the maximal diameter of the maximal axial cross section of the insertion portion 12 may be superior to the diameter of the withdrawal portion 14, according to an axial cross section.

In another embodiment of the present invention, the ratio between the minimal diameter and the maximal diameter of the maximal axial cross section of the insertion portion 12, according to an axial cross section, ranges from 1:1 to 1:4; advantageously from 1:1.5 to 1:2.5; preferably from 1:1.7 to 1:2.0.

These ratios between the minimal diameter and the maximal diameter are one of the parameters defining the elliptical shape of the insertion portion 12 axial cross section. These diameter ratios are believed to be well adapted to the vaginal anatomy and would help prevent leakage and may increase the wearing comfort of the shaped tampon 10.

In an embodiment of the present invention at least two, preferably at least four, of the grooves 22 present on the insertion portion 12 are offset from the grooves 22 present on the withdrawal portion 14.

According to this configuration, a difference in the groove pattern of the insertion portion and the withdrawal portion may be created which would participate to a discontinuity, e.g. truncated groove 22' and bifurcated groove 22" in the overall groove pattern at the shoulder location. It is believed to contribute to the longitudinal flexibility of the tampon according to the present invention, thus increasing the wearing comfort of the user.

In a particular embodiment of the present invention, at least four grooves present on the insertion portion may be contained within a first set of at least two longitudinal cross section planes and at least four grooves present on the withdrawal portion may be contained within a second set of at least two longitudinal cross section planes, wherein these at least four longitudinal cross section planes may be distinct from each other.

In other words, the first set of longitudinal cross section planes, as defined above, may not contain a groove located on the withdrawal portion. The second set of longitudinal cross section planes, as defined above, may not contain a groove located on the withdrawal portion.

This particular setup may participate to the discontinuity in the groove pattern at the shoulder location. It is believed to contribute to the longitudinal flexibility of the tampon according to the present invention, thus increasing the wearing comfort of the user.

In another embodiment of the present invention, the number of grooves on the insertion portion is from three to twelve, advantageously from six to ten, more preferably eight.

In another embodiment of the present invention, the number of grooves on the withdrawal portion is from three to twelve, advantageously from six to ten, more preferably eight.

In a particular configuration, the number of grooves on the insertion portion may be the same as the number of grooves on the withdrawal portion.

As an alternative, the number of grooves on the insertion portion may be different from the number of grooves on the withdrawal portion.

In one preferred embodiment, the number of grooves on the insertion portion may be four and the number of grooves on the withdrawal portion may be four.

In another preferred configuration of the present invention, the number of grooves on the insertion portion may be eight and the number of grooves on the withdrawal portion may be eight.

Grooves 22 may be created by the digital tampon manufacturing process when the tampon pledged is compressed by press jaws as described above. This compression creates a column strength required to impart longitudinal rigidity to the tampon pledget, allowing it to be digitally inserted in the vaginal cavity. The grooves 22 are also believed to help direct the menstrual fluid and help prevent leakage. The number of grooves 22 may be dictated by the manufacturing process and the diameter of the tampon 10.

Thus, the number of grooves 22 may reflect a difference in rigidity for each portion of a tampon or a difference in the fluid absorption properties of each portion. Grooves may also be a distinctive feature of the tampon pledget, creating a pattern that is recognized by the consumer and helping to differentiate from competitor's tampons.

In an embodiment of the present invention, at least two grooves 22 on the insertion portion 12 converge each respectively to one groove 22 on the withdrawal portion 14, transitioning through the shoulder located on the central portion 26 from one radial angle α₁ to another radial angle α₂ (as shown in Figure 6) to form an offset groove.

In other words, there may be at least two grooves 22 that extend longitudinally from one end of the tampon pledget to the other end, through both insertion portion 12, shoulder 42 and withdrawal portion 14.

In a particular configuration of the invention, four grooves 22 on the insertion portion 12 may converge each respectively to one groove 22 on the withdrawal portion 14, through the shoulder located on the central portion 26.

In an embodiment of the present invention, at least two pairs of grooves 22" on the insertion portion converge each respectively to one groove 22" on the withdrawal portion 14 through the shoulder 42 located on the central portion 26.

In other words, the groove pattern on the shaped tampon may present at least a pair of bifurcated grooves 22" having a "Y" shape. Two grooves on the insertion portion may converge with one groove on the withdrawal portion. This pattern may be repeated at least twice to provide a pair, or more, of bifurcated grooves 22".

It should be noted that the word "converging" relates to the direction, not to the depth, of the groove. The surface appearance of the converging grooves of the shaped tampon according to the present invention may be continuous but the actual depth of the grooves on each portion may be similar or different and the converging groove would be less deep at the shoulder location that it is on the insertion or withdrawal portions.

An example of a bifurcated groove according to the invention can be seen in Figure 13.

In a particular configuration of the invention, the at least two bifurcated grooves may be symmetrical, one on each side of the shaped tampon.

Furthermore, the shaped tampon may have two symmetrical bifurcated grooves, one on each side of the tampon.

This very particular construction may participate to the discontinuity in the groove pattern at the shoulder location. It is believed to contribute to the longitudinal flexibility of the tampon according to the present invention. By longitudinal flexibility it is meant that this specific groove pattern may create an ankle at about the shoulder position. This flexibility of the tampon pledget may be an advantage for the consumer as it would create extra comfort but also it would not interfere with the longitudinal column strength that is required for digital insertion.

In another embodiment, the two grooves on the withdrawal portion that are respectively converging to two pairs of grooves on the insertion portion are located in a plane that is defined by two axes, the longitudinal axis and the ellipse minor axis, of the maximal axial cross section of the insertion portion.

In other words, the bifurcated grooves, or "Y" shaped grooves, may be located on the large sides of the shaped tampon as can been seen in Figure 13.

In a particular embodiment, the present invention has at least one truncated groove 22' on the withdrawal portion 14 that does not converge to any groove on the insertion portion 12.

In a preferred configuration, at least two grooves 22' on the withdrawal portion 14 do not converge to any groove 22 on the insertion portion 12.

The at least two truncated grooves 22' on the withdrawal portion 14 may be located in a plane that is defined by two axes, the longitudinal axis and the ellipse major axis, of the maximal axial cross section of the insertion portion.

It is another possibility according to the present invention that none of the grooves on the insertion portion may be located in a plane defined by two axes, the longitudinal axis and the ellipse major axis, of the maximal axial cross section of the insertion portion.

In another configuration, none of the grooves on the insertion portion may be located in a plane that is defined by two axes, the longitudinal axis and the ellipse minor axis, of the maximal axial cross section of the insertion portion.

In an embodiment of the present invention, the grooves 22 on the insertion portion 12 are about evenly distributed.

The grooves 22 on the withdrawal portion 14 may as well be about evenly distributed.

The term "about evenly" should be understood as a regular, or equivalent, spacing between each pair of adjacent grooves with a 0.2 mm, preferably 0.1 mm error margin.

Alternatively, the grooves 22 on the insertion portion 12 may as well be unevenly distributed.

An even distribution of the grooves 22 may be advantageous to achieve a homogenous compression of the fibers but, in some cases, the opposite (i.e. uneven distribution) might be desirable to create different compression zones in the same tampon pledget. This might affect the column strength of the tampon and its absorbency properties (capacity, speed).

In a particular embodiment of the present invention, the fiber density of the insertion portion is superior or equal to the fiber density of the withdrawal portion.

In a more precise configuration, the fiber density of the insertion portion may be within 80% to 120% of the fiber density of the withdrawal portion. Alternatively, the fiber density of the insertion portion may be within 120% to 200% of the fiber density of the withdrawal portion.

This homogeneity in fiber density along the length of the shaped tampon pledget according to the present invention can be achieved by modifying the amount of raw material in the construction of the tampon blank before compression.

Having a homogenous density may provide an even fluid absorbency along the tampon pledget; but an insertion portion with a higher fiber density may contribute to a higher expanding portion on top of the tampon and thus most of the absorption would occur in an area where the vagina is less sensitive, increasing consumer wearing comfort.

The present invention also relates to the use of a shaped tampon according to any of the embodiments disclosed above for collecting catamenial fluids.

The specification and embodiments above are presented to aid in the complete and non-limiting understanding of the invention disclosed herein. Since many variations and embodiments of the invention can be made without departing from its scope, the invention resides in the claims hereinafter appended.

## Claims

1. A press for shaping an absorbent tampon blank into a shaped tampon pledget, said press comprising: a central cavity arranged and configured to accommodate an absorbent tampon blank having a front portion and a back portion; the main longitudinal axis of the press passing in the middle of the central cavity; a plurality of penetrating dies, each penetrating die having a pressing face on at least a fragment of its side facing the central cavity and the plurality of penetrating dies being arranged radially around the said main longitudinal axis; and at least one cam to actuate the plurality of penetrating dies, wherein a plurality of the said penetrating dies are divided longitudinally, creating a group of composite penetrating dies, said composite penetrating dies having two parts, a front part and a back part, and all the front parts of the composite penetrating dies constitute the front group and all the back parts of the composite penetrating dies constitute the back group, wherein the front group and the back group are actuated independently,
wherein:
- at least one penetrating die having a pressing face with a first shaped longitudinal profile, and
- at least one penetrating die having a pressing face with a second shaped longitudinal profile.

2. A press according to claim 1, wherein all the penetrating dies are composite penetrating dies, and/or wherein at least two composite penetrating die front parts are identical, and/or wherein at least two composite penetrating die back parts are identical.

3. A press according to claim 1 or 2, wherein at least two of the composite penetrating dies have back part and front part pressing faces that are positioned to produce, on the outer surface of an absorbent tampon pledget, a substantially continuous groove made of two segments, the extremities of the two pressing faces being adjacent at a connection point, and/or wherein a plurality of the composite penetrating die back parts have pressing faces that are not parallel to the main longitudinal axis, and/or the pressing faces are helically arranged.

4. A press according to any of claims 1 to 3, wherein at least two adjacent composite penetrating dies have front part and back part pressing faces that have no connection point.

5. A press according to any of claims 1 to 4, wherein the pressing faces of at least two adjacent composite penetrating die front parts and the pressing face of one composite penetrating die back part are positioned to produce, on the outer surface of an absorbent tampon pledget, a bifurcated groove made of three segments, the extremities of said three pressing faces being adjacent at a connection point.

6. A press according to any of claims 1 to 5 wherein at least 2 of the composite penetrating die front parts have pressing faces with a shaped longitudinal profile, and/or wherein at least 4 of the composite penetrating die front parts have pressing faces with a shaped longitudinal profile, and/or wherein at least 6 of the composite penetrating die front parts have pressing faces with a shaped longitudinal profile, and/or wherein at least 8 of the composite penetrating die front parts have pressing faces with a shaped longitudinal profile.

7. A press according to claim 3 or any claim dependent thereon, wherein the connection points of
- the two pressing faces of the composite penetrating dies positioned to produce a substantially continuous groove made of two segments, and
- the three pressing faces of the composite penetrating dies positioned to produce a bifurcated groove made of three segments,
are not all included in the same radial plane.

8. A method for producing a shaped tampon pledget comprising the steps of
- introducing a tampon blank in a press according to any of the preceding claims,
- actuating a first group of penetrating dies to compress the tampon blank with a first set of pressing faces,
- actuating a second group of penetrating dies to compress the tampon blank with a second set of pressing faces,
- partially disengaging one of the groups of penetrating dies to move the corresponding pressing faces into a transfer position, thus allowing the front portion of a compressed tampon pledget to pass through said partially disengaged group of penetrating dies,
- partially disengaging the second group of penetrating dies, and
- ejecting a resulting compressed tampon pledget from the press to a carrier device, optionally wherein the first group of penetrating dies are the composite penetrating die front parts and the second group of penetrating dies are the composite penetrating die back parts according to claim 2 or any claim dependent thereon.

9. A shaped tampon having a longitudinal axis, an insertion portion with an insertion tip, a withdrawal portion with a withdrawal end, the insertion and withdrawal portions being connected through a central portion and each portion comprising a plurality of grooves,
**characterized by**
• the contour of said insertion portion in an axial cross section plane being an ellipse, the ellipse having a major axis and a minor axis, wherein the major axis is not equal to the minor axis; and a maximal axial cross section,
• the contour of the insertion portion is curved in at least one longitudinal cross section plane, and
• the grooves on the insertion portion extend from the central portion to the insertion tip and are merged in a dome shaped insertion tip.

10. The shaped tampon according to claim 9, wherein the longitudinal opposite sides of the contour of the withdrawal portion according to a longitudinal cross section plane are substantially parallel, and/or the contour of said withdrawal portion according to an axial cross section plane is substantially circular.

11. The shaped tampon according to claim 9 or claim 10, wherein the central portion contains a shoulder at which the insertion portion and withdrawal portion transition.

12. The shaped tampon according to any of claims 9 to 11, wherein the maximal axial cross section of the insertion portion is located, along the longitudinal axis, between the central portion and 10mm from the insertion tip, and/or wherein the minimal diameter of the maximal axial cross section of the insertion portion is equal or inferior to the diameter of the withdrawal portion, according to an axial cross section.

13. The shaped tampon according to any of claims 9 to 12, wherein the ratio between the minimal diameter and the maximal diameter of the maximal axial cross section of the insertion portion, according to an axial cross section, ranges from 1:1 to 1:4, optionally wherein the ratio between the minimal diameter and the maximal diameter of the maximal axial cross section of the insertion portion, according to an axial cross section, ranges from 1:1.5 to 1:2.5, further optionally wherein the ratio between the minimal diameter and the maximal diameter of the maximal axial cross section of the insertion portion, according to an axial cross section, ranges from 1:1.7 to 1:2.0.

14. The shaped tampon according to any of claims 9 to 13, wherein at least two of the grooves present on the the insertion portion are offset from the grooves present on the withdrawal portion, optionally wherein at least four of the grooves present on the insertion portion are offset from the grooves present on the withdrawal portion.

15. The shaped tampon according to any of claims 9 to 14, wherein the number of grooves on the insertion portion is from 3 to 12 and the number of grooves on the withdrawal portion is from 3 to 12, and/or 6 to 10, and/or 8, and/or wherein the number of grooves on the withdrawal portion is from 6 to 10, or wherein the number of grooves on the withdrawal portion is 8.

16. The shaped tampon according to claim 11 or any claim dependent thereon, wherein at least 2 grooves on the insertion portion converge each respectively to one groove on the withdrawal portion, through the shoulder located on the central portion, and/or at least 2 pairs of grooves on the insertion portion converge each respectively to 1 groove on the withdrawal portion through the shoulder located on the central portion, and/or 2 grooves on the withdrawal portion that respectively converge to 2 pairs of grooves on the insertion portion are located in a plane that is defined by 2 axes:
- the longitudinal axis and
- the ellipse minor axis of the maximal axial cross section of the insertion portion, and/or wherein at least one groove on the withdrawal portion does not converge to any groove on the insertion portion.

17. The shaped tampon according to any of claims 9 to 16, wherein the grooves on the insertion portion are about evenly distributed.

18. The shaped tampon according to any of claims 9 to 17, wherein the fiber density of the insertion portion is superior or equal to the fiber density of the withdrawal portion.

## Patentansprüche

1. Presse zum Formen eines saugfähigen Tamponrohlings zu einem geformten Tamponbausch, wobei die Presse umfasst: einen zentralen Hohlraum, der dazu angeordnet und ausgelegt ist, einen saugfähigen Tamponrohling mit einem vorderen Abschnitt und einem hinteren Abschnitt aufzunehmen, wobei die Hauptlängsachse der Presse in der Mitte des zentralen Hohlraums verläuft, eine Vielzahl von Eindringmatrizen, wobei jede Eindringmatrize eine Druckfläche auf mindestens einem Fragment ihrer dem zentralen Hohlraum zugewandten Seite hat und wobei die Vielzahl von Eindringmatrizen radial um die Hauptlängsachse angeordnet ist; und mindestens einen Nocken, um die Vielzahl von Eindringmatrizen zu betätigen, wobei eine Vielzahl der Eindringmatrizen in Längsrichtung unterteilt ist und eine Gruppe von Verbundeindringmatrizen bildet, wobei die Verbundeindringmatrizen zwei Teile haben, ein vorderes Teil und ein hinteres Teil, und alle vorderen Teile der Verbundeindringmatrizen die vordere Gruppe bilden und alle hinteren Teile der Verbundeindringmatrizen die hintere Gruppe bilden, wobei die vordere Gruppe und die hintere Gruppe unabhängig betätigt werden,
wobei:
- mindestens eine Eindringmatrize eine Druckfläche mit einem ersten geformten Längsprofil hat, und
- mindestens eine Eindringmatrize eine Druckfläche mit einem zweiten geformten Längsprofil hat.

2. Presse nach Anspruch 1, wobei alle Eindringmatrizen Verbundeindringmatrizen sind, und/oder wobei mindestens zwei vordere Teile der Verbundeindringmatrize identisch sind, und/oder wobei mindestens zwei hintere Teile der Verbundeindringmatrize identisch sind.

3. Presse nach Anspruch 1 oder 2, wobei mindestens zwei der Verbundeindringmatrizen Druckflächen der hinteren Teile und der vorderen Teile haben, die dazu positioniert sind, auf der Außenseite eines saugfähigen Tamponbausches eine im Wesentlichen durchgehende Rille aus zwei Segmenten zu produzieren, wobei die Gliedmaßen der zwei Druckflächen neben einem Verbindungspunkt liegen, und/oder wobei eine Vielzahl der hinteren Teile der Verbundeindringmatrize Druckflächen hat, die nicht parallel zu der Hauptlängsachse sind, und/oder die Druckflächen schraubenförmig angeordnet sind.

4. Presse nach einem der Ansprüche 1 bis 3, wobei mindestens zwei benachbarte Verbundeindringmatrizen Druckflächen der vorderen Teile und der hinteren Teile haben, die keinen Verbindungspunkt haben.

5. Presse nach einem der Ansprüche 1 bis 4, wobei die Druckflächen der mindestens zwei benachbarten vorderen Teile der Verbundeindringmatrize und die Druckfläche von einem hinteren Teil einer Verbundeindringmatrize dazu positioniert sind, auf der Außenseite eines saugfähigen Tamponbausches eine verzweigte Rille aus drei Segmenten zu produzieren, wobei die Gliedmaßen der drei Druckflächen neben einem Verbindungspunkt liegen.

6. Presse nach einem der Ansprüche 1 bis 5, wobei mindestens 2 der vorderen Teile der Verbundeindringmatrize Druckflächen mit einem geformten Längsprofil haben, und/oder wobei mindestens 4 der vorderen Teile der Verbundeindringmatrize Druckflächen mit einem geformten Längsprofil haben, und/oder wobei mindestens 6 der vorderen Teile der Verbundeindringmatrizen Druckflächen mit einem geformten Längsprofil haben, und/oder wobei mindestens 8 der vorderen Teile der Verbundeindringmatrizen Druckflächen mit einem geformten Längsprofil haben.

7. Presse nach Anspruch 3 oder einem davon abhängigen Anspruch, wobei die Verbindungspunkte
- der zwei Druckflächen der Verbundeindringmatrizen, die dazu positioniert sind, eine im Wesentlichen durchgehende Rille aus zwei Segmenten zu produzieren, und
- der drei Druckflächen der Verbundeindringmatrizen, die dazu positioniert sind, eine verzweigte Rille aus drei Segmenten zu produzieren,
nicht alle in derselben Radialebene enthalten sind.

8. Verfahren zum Produzieren eines geformten Tamponbausches, umfassend die Schritte
- Einführen eines Tamponrohlings in eine Presse nach einem der vorhergehenden Ansprüche,
- Betätigen einer ersten Gruppe von Eindringmatrizen, um den Tamponrohling mit einem ersten Satz von Druckflächen zu komprimieren,
- Betätigen einer zweiten Gruppe von Eindringmatrizen, um den Tamponrohling mit einem zweiten Satz von Druckflächen zu komprimieren,
- teilweises Lösen von einer der Gruppen von Eindringmatrizen, um die entsprechenden Druckflächen in eine Übergangsposition zu bewegen, was ermöglicht, das der vordere Abschnitt eines komprimierten Tamponbausches durch die teilweise gelöste Gruppe von Eindringmatrizen durchgeht,
- teilweises Lösen der zweiten Gruppe von Eindringmatrizen, und
- Auswerfen eines resultierenden komprimierten Tamponbausches aus der Presse in eine Trägervorrichtung, wobei optional die erste Gruppe von Eindringmatrizen die vorderen Teile der Verbundeindringmatrize bildet und die zweite Gruppe von Eindringmatrizen die hinteren Teile der Verbundeindringmatrize nach Anspruch 2 oder einem davon abhängigen Anspruch bildet.

9. Geformter Tampon mit einer Längsachse, einem Einführabschnitt mit einer Einführspitze, einem Rückholabschnitt mit einem Rückholende, wobei der Einführabschnitt und der Rückholabschnitt über einen zentralen Abschnitt verbunden sind, und jeder Abschnitt eine Vielzahl von Rillen umfasst,
**gekennzeichnet dadurch, dass**
• die Kontur des Einführabschnitts in einer axialen Querschnittsebene eine Ellipse ist, wobei die Ellipse eine Hauptachse und eine Nebenachse hat, wobei die Hauptachse nicht gleich der Nebenachse ist, und **gekennzeichnet durch** einen maximalen axialen Querschnitt,
• die Kontur des Einführabschnitts in mindestens eine Längsquerschnittsebene gekrümmt ist, und
• sich die Rillen an dem Einführabschnitt von dem zentralen Abschnitt zu der Einführspitze erstrecken und in einer kuppelförmigen Einführspitze zusammenlaufen.

10. Geformter Tampon nach Anspruch 9, wobei die gegenüberliegenden Längsseiten der Kontur des Rückholabschnitts gemäß einer Längsquerschnittsebene im Wesentlichen parallel sind, und/oder die Kontur des Rückholabschnitts gemäß einer axialen Querschnittsebene im Wesentlichen kreisförmig ist.

11. Geformter Tampon nach Anspruch 9 oder Anspruch 10, wobei der zentrale Abschnitt eine Schulter enthält, an der der Einführabschnitt und der Rückholabschnitt wechseln.

12. Geformter Tampon nach einem der Ansprüche 9 bis 11, wobei sich der maximale axiale Querschnitt des Einführabschnitts entlang der Längsachse zwischen dem zentralen Abschnitt und 10mm von der Einführspitze befindet, und/oder wobei der minimale Durchmesser des maximalen axialen Querschnitts des Einführabschnitts gleich oder geringer ist als der Durchmesser des Rückholabschnitts gemäß einem axialen Querschnitt.

13. Geformter Tampon nach einem der Ansprüche 9 bis 12, wobei das Verhältnis zwischen dem Mindestdurchmesser und dem Maximaldurchmesser des maximalen axialen Querschnitts des Einführabschnitts, gemäß einem axialen Querschnitt, von 1:1 bis 1:4 reicht, wobei optional das Verhältnis zwischen dem minimalen Durchmesser und dem maximalen Durchmesser des maximalen axialen Querschnitts des Einführabschnitts, gemäß einem axialen Querschnitt, von 1:1,5 bis 1:2,5 reicht, wobei ferner optional das Verhältnis zwischen dem minimalen Durchmesser und dem maximalen Durchmesser des maximalen axialen Querschnitts des Einführabschnitts, gemäß einem axialen Querschnitt, von 1:1,7 bis 1:2,0 reicht.

14. Geformter Tampon nach einem der Ansprüche 9 bis 13, wobei mindestens zwei der an dem Einführabschnitt vorhandenen Rillen von den auf dem Rückholabschnitt vorhandenen Rillen versetzt sind, wobei optional mindestens vier der auf dem Einführabschnitt vorhandenen Rillen von den auf dem Rückholabschnitt vorhandenen Rillen versetzt sind.

15. Geformter Tampon nach einem der Ansprüche 9 bis 14, wobei die Anzahl an Rillen an dem Einführabschnitt von 3 bis 12 beträgt und die Anzahl an Rillen an dem Rückholabschnitt von 3 bis 12 beträgt, und/oder 6 bis 10, und/oder 8, und/oder wobei die Anzahl an Rillen an dem Rückholabschnitt von 6 bis 10 beträgt, oder wobei die Anzahl an Rillen an dem Rückholabschnitt 8 beträgt.

16. Geformter Tampon nach Anspruch 11 oder einem davon abhängigen Anspruch, wobei sich mindestens 2 Rillen an dem Einführabschnitt jeweils über die an dem zentralen Abschnitt befindliche Schulter mit einer Rille an dem Rückholabschnitt konvergieren, und/oder mindestens 2 Paare von Rillen an dem Einführabschnitt jeweils mit 1 Rille an dem Rückholabschnitt über die an dem zentralen Abschnitt befindliche Schulter konvergieren, und/oder 2 Rillen an dem Rückholabschnitt, die jeweils mit 2 Paaren von Rillen an dem Einführabschnitt konvergieren, in einer Ebene befinden, die von 2 Achsen definiert ist:
- der Längsachse und
- der Ellipsennebenachse des maximalen axialen Querschnitts des Einführabschnitts, und/oder wobei mindestens eine Rille an dem Rückholabschnitt mit keiner Rille an dem Einführabschnitt konvergiert.

17. Geformter Tampon nach einem der Ansprüche 9 bis 16, wobei die Rillen an dem Einführabschnitt etwa gleichmäßig verteilt sind.

18. Geformter Tampon nach einem der Ansprüche 9 bis 17, wobei die Faserdichte des Einführabschnitts höher oder gleich der Faserdichte des Rückholabschnitts ist.

## Revendications

1. Presse pour le façonnage d'une ébauche de tampon absorbant en une compresse de tampon façonnée, ladite presse comprenant : une cavité centrale agencée et configurée pour recevoir une ébauche de tampon absorbant ayant une partie avant et une partie arrière ; l'axe longitudinal principal de la presse passant au milieu de la cavité centrale ; une pluralité de matrices pénétrantes, chaque matrice pénétrante ayant une face de pressage sur au moins un fragment de son côté faisant face à la cavité centrale et la pluralité de matrices pénétrantes étant agencées radialement autour dudit axe longitudinal principal ; et au moins une came pour actionner la pluralité de matrices pénétrantes, une pluralité desdites matrices pénétrantes étant divisées longitudinalement, créant un groupe de matrices pénétrantes composites, lesdites matrices pénétrantes composites ayant deux parties, une partie avant et une partie arrière, et toutes les parties avant des matrices pénétrantes composites constituant le groupe avant et toutes les parties arrière des matrices pénétrantes composites constituant le groupe arrière, le groupe avant et le groupe arrière étant actionnés de manière indépendante,
- au moins une matrice pénétrante ayant une face de pressage avec un premier profil longitudinal façonné, et
- au moins une matrice pénétrante ayant une face de pressage avec un second profil longitudinal façonné.

2. Presse selon la revendication 1, toutes les matrices pénétrantes étant des matrices pénétrantes composites, et/ou au moins deux parties avant de matrice pénétrante composite étant identiques, et/ou au moins deux parties arrière de matrice pénétrante composite étant identiques.

3. Presse selon la revendication 1 ou 2, au moins deux des matrices pénétrantes composites ayant des faces de pressage de partie arrière et de partie avant qui sont positionnées pour produire, sur la surface extérieure d'une compresse de tampon absorbant, une rainure sensiblement continue composée de deux segments, les extrémités des deux faces de pressage étant adjacentes à un point de connexion, et/ou une pluralité des parties arrière de matrice pénétrante composite ayant des faces de pressage qui ne sont pas parallèles à l'axe longitudinal principal, et/ou les faces de pressage étant agencées de manière hélicoïdale.

4. Presse selon l'une quelconque des revendications 1 à 3, au moins deux matrices pénétrantes composites adjacentes ayant des faces de pressage de partie avant et de partie arrière qui n'ont pas de point de connexion.

5. Presse selon l'une quelconque des revendications 1 à 4, les faces de pressage d'au moins deux parties avant de matrice pénétrante composite adjacentes et la face de pressage d'une partie arrière de matrice pénétrante composite étant positionnées de manière à produire, sur la surface extérieure d'une compresse de tampon absorbant, une rainure bifurquée composée de trois segments, les extrémités desdites trois faces de pressage étant adjacentes à un point de connexion.

6. Presse selon l'une quelconque des revendications 1 à 5, au moins 2 des parties avant de matrice pénétrante composite ayant des faces de pressage avec un profil longitudinal façonné, et/ou au moins 4 des parties avant de matrice pénétrante composite ayant des faces de pressage avec un profil longitudinal façonné, et/ou au moins 6 des parties avant de matrice pénétrante composite ayant des faces de pressage avec un profil longitudinal façonné, et/ou au moins 8 des parties avant de matrice pénétrante composite ayant des faces de pressage avec un profil longitudinal façonné.

7. Presse selon la revendication 3 ou toute revendication qui en dépend, les points de connexion
- des deux faces de pressage des matrices pénétrantes composites positionnées de manière à produire une rainure substantiellement continue composée de deux segments, et
- des trois faces de pressage des matrices pénétrantes composites positionnées pour produire une rainure bifurquée composée de trois segments,
n'étant pas toutes comprises dans le même plan radial.

8. Procédé de fabrication d'une compresse de tampon façonnée comprenant les étapes :
- d'introduction d'une ébauche de tampon dans une presse selon l'une quelconque des revendications précédentes,
- d'actionnement d'un premier groupe de matrices pénétrantes pour comprimer l'ébauche de tampon avec un premier ensemble de faces de pressage,
- d'actionnement d'un deuxième groupe de matrices pénétrantes pour comprimer l'ébauche de tampon avec un deuxième ensemble de faces de pressage,
- de désengagement partiel de l'un des groupes de matrices pénétrantes pour placer les faces de pressage correspondantes dans une position de transfert, permettant ainsi à la partie avant d'une compresse de tampon comprimé de passer à travers ledit groupe de matrices pénétrantes partiellement désengagé,
- de désengagement partiel du deuxième groupe de matrices pénétrantes, et
- d'éjection d'une compresse de tampon comprimé résultante de la presse vers un dispositif de transport, éventuellement le premier groupe de matrices pénétrantes étant les parties avant de matrice pénétrante composite et le deuxième groupe de matrices pénétrantes étant les parties arrière de matrice pénétrante composite selon la revendication 2 ou toute autre revendication qui en dépend.

9. Tampon façonné ayant un axe longitudinal, une partie d'insertion avec une pointe d'insertion, une partie de retrait avec une extrémité de retrait, les parties d'insertion et de retrait étant reliées par une partie centrale et chaque partie comprenant une pluralité de rainures,
**caractérisé en ce que**
• le contour de ladite partie d'insertion dans un plan de section transversale axiale est une ellipse, l'ellipse ayant un grand axe et un petit axe, le grand axe n'étant pas égal au petit axe ; et une section transversale axiale maximale,
• le contour de la partie d'insertion étant incurvé dans au moins un plan de section transversale longitudinale, et
• les rainures de la partie d'insertion s'étendant de la partie centrale à la pointe d'insertion et se rejoignent en une pointe d'insertion en forme de dôme.

10. Tampon façonné selon la revendication 9, les côtés longitudinaux opposés du contour de la partie de retrait selon un plan de section transversale longitudinale étant sensiblement parallèles, et/ou le contour de ladite partie de retrait selon un plan de section transversale axiale étant sensiblement circulaire.

11. Tampon façonné selon la revendication 9 ou la revendication 10, la partie centrale contenant un épaulement au niveau duquel la partie d'insertion et la partie de retrait sont en transition.

12. Tampon façonné selon l'une quelconque des revendications 9 à 11, la section transversale axiale maximale de la partie d'insertion étant située, le long de l'axe longitudinal, entre la partie centrale et 10 mm de la pointe d'insertion, et/ou le diamètre minimal de la section transversale axiale maximale de la partie d'insertion étant égal ou inférieur au diamètre de la partie de retrait, selon une section transversale axiale.

13. Tampon façonné selon l'une quelconque des revendications 9 à 12, le rapport entre le diamètre minimal et le diamètre maximal de la section transversale axiale maximale de la partie d'insertion, selon une section transversale axiale, étant dans la plage de 1:1 à 1:4, éventuellement le rapport entre le diamètre minimal et le diamètre maximal de la section transversale axiale maximale de la partie d'insertion, selon une section transversale axiale, étant dans la plage de 1:1,5 à 1:2,5, et éventuellement le rapport entre le diamètre minimal et le diamètre maximal de la section transversale axiale maximale de la partie d'insertion, selon une section transversale axiale, étant dans la plage de 1:1,7 à 1:2,0.

14. Tampon façonné selon l'une quelconque des revendications 9 à 13, au moins deux des rainures présentes sur la partie d'insertion étant décalées par rapport aux rainures présentes sur la partie de retrait, éventuellement au moins quatre des rainures présentes sur la partie d'insertion étant décalées par rapport aux rainures présentes sur la partie de retrait.

15. Tampon façonné selon l'une quelconque des revendications 9 à 14, le nombre de rainures sur la partie d'insertion étant compris entre 3 et 12 et le nombre de rainures sur la partie de retrait étant compris entre 3 et 12, et/ou 6 et 10, et/ou 8, et/ou le nombre de rainures sur la partie de retrait étant compris entre 6 et 10, ou le nombre de rainures sur la partie de retrait étant de 8 .

16. Tampon façonné selon la revendication 11 ou toute revendication qui en dépend, au moins 2 rainures sur la partie d'insertion convergeant chacune respectivement vers une rainure sur la partie de retrait, à travers l'épaulement situé sur la partie centrale, et/ou au moins 2 paires de rainures sur la partie d'insertion convergeant chacune respectivement vers 1 rainure sur la partie de retrait à travers l'épaulement situé sur la partie centrale, et/ou 2 rainures sur la partie de retrait qui convergent respectivement vers 2 paires de rainures sur la partie d'insertion étant situées dans un plan qui est défini par 2 axes :
- l'axe longitudinal et
- l'axe mineur de l'ellipse de la section transversale axiale maximale de la partie d'insertion, et/ou au moins une rainure de la partie de retrait ne convergeant vers aucune rainure de la partie d'insertion.

17. Tampon façonné selon l'une quelconque des revendications 9 à 16, les rainures de la partie d'insertion étant à peu près uniformément réparties.

18. Tampon façonné selon l'une quelconque des revendications 9 à 17, la densité des fibres de la partie d'insertion étant supérieure ou égale à la densité des fibres de la partie de retrait.
